# EUROPÄISCHE PATENTSCHRIFT

(11) **EP 1 318 404 B1**
(45) Veröffentlichungstag und Bekanntmachung des Hinweises auf die Patenterteilung: **28.07.2004**
(21) Anmeldenummer: 01128850.3
(22) Anmeldetag: 04.12.2001
(51) Int. Cl.: G01N 33/68, G01N 33/569

(54) **Verfahren zur Diagnose von Sepsis unter Bestimmung von CA 19-9**
Diagnosis of sepsis determining CA 19-9
Diagnostic de la septicémie par détermination de la CA 19-9

(43) Veröffentlichungstag der Anmeldung: 11.06.2003
(73) Patentinhaber: B.R.A.H.M.S Aktiengesellschaft, 16761 Hennigsdorf (DE)
(72) Erfinder: Bergmann, Andreas, Dr., 12351 Berlin (DE)
(74) Vertreter: Andrae, Steffen, Dr.

(56) Entgegenhaltungen:
- PFISTER MARC ET AL: "Elevated carbohydrate antigen 19-9 (CA 19-9) in patients with Echinococcus infection." CLINICAL CHEMISTRY AND LABORATORY MEDICINE, Bd. 39, Nr. 6, Juni 2001 (2001-06), Seiten 527-530, XP008003114 ISSN: 1434-6621
- KER C-G ET AL: "ASSESSMENT OF SERUM AND BILE LEVELS OF CA19-9 AND CA125 IN CHOLANGITIS AND BILE DUCT CARCINOMA" JOURNAL OF GASTROENTEROLOGY AND HEPATOLOGY, Bd. 6, Nr. 5, 1991, Seiten 505-508, XP008003115 ISSN: 0815-9319
- REINHART, K ET AL: "Intensivmedizin: Sepsis und septischer Schock (Seiten 756-760)" 2001 , THIEME VERLAG, XP008003130 * Seite 756, rechte Spalte, Absatz 3 *
- BEISHUIZEN A ET AL: "Endogenous mediators in sepsis and septic shock" ADVANCES IN CLINICAL CHEMISTRY, Bd. 33, 1999, Seiten 55-131, XP008003176

## Beschreibung

Die Erfindung betrifft ein neues Verfahren zur Sepsisdiagnose, bei dem oder im Rahmen dessen der an sich in der medizinischen Diagnostik als typischer Tumormarker bekannte Parameter CA 19-9 bestimmt wird.

Sie beruht auf dem erstmaligen Nachweis stark erhöhter Konzentrationen von CA 19-9 in Seren von Patienten, bei denen aufgrund klinischer Befunde bei gleichzeitig erhöhten Serumkonzentrationen des bekannten Sepsismarkers Procalcitonin eine Sepsis diagnostiziert worden war.

Die vorliegende Erfindung hat ihren Ursprung in intensiven Forschungsarbeiten der Anmelderin im Zusammenhang mit weiteren Verbesserungen der Diagnose und Therapie von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Bei Sepsis bzw. dem septische Schock weiten sich entzündungsspezifische Reaktionskaskaden unkontrolliert auf den gesamten Körper aus und können dabei, im Sinne einer überschießenden systemischen Immunantwort, lebensbedrohlich werden. Zu den gegenwärtigen Kenntnissen über das Auftreten und die möglichen Rolle einzelner Gruppen endogener sepsisspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung wird daher der Begriff Sepsis in Anlehnung an die Definitionen verwendet, wie sie den genannten Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß oder vermutet, dass sie an einem Entzündungsgeschehen beteiligt sind, eignen sich dabei für Zwecke der klinischen Sepsisdiagnostik insbesondere solche, die bei Sepsis oder bestimmten Phasen einer Sepsis mit hoher Sensitivität und Spezifität auftreten bzw. deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen mit dem jeweiligen Biomarker im Vordergrund, ohne dass dessen Rolle in der komplexen Kaskade der am Sepsisgeschehen beteiligten endogenen Substanzen genau bekannt sein muss.

Eine bekannte, als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Besonders wertvoll ist die Procalcitoninbestimmung ferner zur therapiebegleitenden Verlaufsbeobachtung einer Sepsis. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig bei der Anmelderin intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. So werden insbesondere weitere Biomarker für die Sepsisdiagnostik gesucht, deren Serum- bzw. Plasmaspiegel zwar regelmäßig erhöht sind, die bei ihrer Bestimmung jedoch nicht einfach die Ergebnisse der Procalcitoninbestimmung duplizieren, sondern zusätzliche Informationen liefern, und zwar insbesondere zum Stadium des Sepsisgeschehens, d.h. eine dem Sepsisverlauf eher zeitlich zuzuordnende Information, und/oder zum Ausgangs- oder Schwerpunktorgan eines septischen Geschehens, d.h. eine lokalisierende Information. Ziel ist letztlich die Selektion eines Satzes von Sepsisparametern, die bei Sepsispatienteri bzw. potentiellen Sepsispatienten gleichzeitig bestimmt werden, z.B. unter Nutzung der sog. Chip-Technologie oder immunchromatographischer Verfahren ("Point of Care" oder POC-Bestimmungen), und dabei in ihrer Gesamtheit ein Informationsmuster liefern, das den Informationswert der Bestimmung nur eines einzelnen Parameters klar übertrifft.

Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Da die bei Sepsis erhöhten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Sepsisgeschehen einzugreifen, um zum Beispiel die bei Sepsis beobachtete systemische Ausbreitung der Entzündung möglichst frühzeitig zu stoppen. In diesem Sinne sind nachweislich am Sepsisgeschehen beteiligte endogene Substanzen auch als potentielle therapeutische Targets anzusehen.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin signifikant erhöht ist, sondern auch für andere Substanzen, die zu den Peptid-Prohormonen gerechnet werden können, signifikant erhöhte Konzentrationen beobachtet werden können. Es sind in diesem Zusammenhang insbesondere zu nennen die Peptid-Prohormone pro-Enkephalin, pro-Gastrin-Releasing Peptid (proGRP), pro-Endothelin-1, pro-Brain-Natriuretic-Peptid (pro-BNP), pro-Atrial-Natriuretic-Peptid (pro-ANP), pro-Leptin, pro-Neuropeptid-Y, pro-Somatostatin, pro-Neuropeptid-YY, pro-Interleukin 6 oder pro-Interleukin-10. Während das beschriebene Phänomen gut dokumentiert ist, sind die Ursachen für den Anstieg der Konzentrationen von Prohormonen bei Sepsis weiterhin weitgehend ungeklärt.

In der vorliegenden Anmeldung wird nunmehr ein Ergebnis eines anderen hypothetischen Ansatzes für die Suche nach weiteren für die Sepsisdiagnostik geeigneten Biomolekülen berichtet. Sie beruht auf den Ergebnissen von Messungen der physiologischen Konzentrationen von Biomarkern, die bisher als typische Tumormarker angesehen und daher klinisch im wesentlichen zu Zwecken der Tumordiagnostik bestimmt wurden, in biologischen Proben, insbesondere Serumproben, von Sepsispatienten, bei denen keinerlei klinische Befunde auf das Vorliegen von Tumoren hinwiesen.

Überraschenderweise zeigte sich, dass bei Sepsis auch einige bisher als typische Tumormarker angesehene Biomoleküle signifikant erhöht sind. Das deutet darauf hin, dass diese nicht tumorspezifisch gebildet werden, sondern eher ein physiologisches Krisengeschehen anzeigen, das auch Gewebe bzw. Organe erfaßt, die diese Tumormarker freisetzen. Und obwohl, wie in dieser Anmeldung und gleichzeitig eingereichten weiteren Anmeldungen gezeigt wird, die Konzentrationen der fraglichen Biomoleküle bei Sepsis mit hoher Sensitivität erhöht sind, besteht gleichzeitig keine Korrelation der gemessenen Werte zu den ebenfalls signifikant erhöhten Procalcitoninkonzentrationen, d.h. bei einzelnen Patienten werden zwar beide Parameter erhöht gefunden, jedoch teilweise in ganz unterschiedlicher Ausprägung.

Die vorliegende Erfindung beruht auf dem erstmaligen Nachweis, dass bei bakterieller Sepsis signifikant erhöhte physiologische Konzentrationen CA 19-9 gefunden werden, was diesen Parameter, insbesondere in Kombination mit der Bestimmung weiterer Sepsisparameter, für die differentielle - Sepsisdiagnostik geeignet macht.

Das erfindungsgemäße Verfahren und bestimmte bevorzugte Ausgestaltungen davon sind in den Ansprüchen 1 bis 6 genauer definiert.

Es ist bisher nicht bekannt, dass die messbaren Konzentrationen von CA 19-9, insbesondere Serumkonzentrationen, bei Sepsis signifikant erhöht sind und dass daher eine Bestimmung der Konzentration von CA 19-9 auch für die Sepsisdiagnostik von Bedeutung sein kann.

Aufgrund der vorliegenden Erfindung ist es möglich, die Bestimmung von CA 19-9 auch im Rahmen eines diagnostischen Sepsis-Nachweisverfahrens zu nutzen. Von besonderem Interesse ist die Eignung von CA 19-9 als Prognosemarker und Marker für die Überwachung der Krankheitsverlaufs von Sepsis, insbesondere im Rahmen einer Kombinationsmessung mit anderen Markern.

Neben einer Kombination mit einer Procalcitoninmessung kommt insbesondere eine Kombination des Messung von CA 19-9 mit der Bestimmung anderer Marker für Sepsis und systemische Entzündungen, die bisher als typische Tumormarker angesehen wurden, in Betracht, und zwar insbesondere mit CA 125, S100B oder an der Regulierung von Entzündungen beteiligten S100A-Proteinen, oder mit der Bestimmung der in den älteren, nachfolgend genannten deutschen Patentanmeldungen der Anmelderin beschriebenen neuartigen Sepsismarker Inflammin (DE 101 19 804.3) und CHP (DE 101 31 922.3), und/oder mit der Bestimmung von löslichen Cytokeratinfragmenten, insbesondere der neu aufgefundenen löslichen Cytokeratin-1-Fragmente (sCY1F; DE 101 30 985.6) sowie der bekannten Tumormarker CYFRA-21 oder TPS, und/oder der Bestimmung eines oder mehrerer der o.g. Prohormone. Auch eine gleichzeitige Bestimmung des bekannten Entzündungsparameters C-reaktives Protein (CRP) kann vorgesehen sein. Aufgrund der in dieser und den parallelen Anmeldungen beschriebenen neuen Ergebnisse ist für die Sepsis-Feindiagnose außerdem generell eine Kombination mit Messungen von bekannten oder noch zu findenden Biomolekülen in Betracht zu ziehen, die als gewebe- bzw. organspezifische Entzündungsmarker geeignet sind.

Als CA 19-9 (cancer antigen 19-9) oder GICA (gastrointestinal cancer antigen) wird ein Glykolipid mit einer molaren Masse von ca. 36 kD bezeichnet, das durch Reaktion mit einem bestimmten spezifischen monoklonalen Antikörper (1116 NS 19-9; vgl. Koprowski et al., Somatic Cell Genet 1979, 5:957-972) identifizierbar ist. seine biologische Funktion ist unbekannt. Es spielt aufgrund seiner hohen Spezifität eine wichtige Rolle bei der Diagnostik, Therapie- und Verlaufskontrolle von gastrointestinalen Karzinomen, insbesondere auch beim Pankreaskarzinom, hepatobilären Karzinom (Leberkarzinom, Gallenwegs-Karzinom) und Magenkarzinom. (vgl. Lothar Thomas (Hrsg.): Labor und Diagnose, Kapitel 34.3, S.966-969, 5.Auflage, 1998 TH-Books Verlagsgesellschaft).

Es ist bekannt, dass auch bei einigen Patienten mit nichtmalignen entzündlichen Erkrankungen wie Cholezystitis und obstruktivem Ikterus, Cholelithiasis, Cholezystolithiasis, akuter Cholangitits, toxischer Hepatitis und anderen Lebererkrankungen in Größenordnungen von etwa 20 % der Fälle erhöhte Spiegel von CA 19-9 gemessen werden können (vgl. Lothar Thomas, a.a.O.; M.J. Duffy, Ann Clin Biochem 1998; 35; 364-370). Weiterhin ist in Patienten, die mit Echinococcus infiziert sind, ein erhöhter CA 19-9 Spiegel beschrieben worden (Pfister, M. et al., Clin. Chem. Lab. Med., Vol. 39, S. 527 - 530, 2001).

Bei septischen Patienten wurden nach diesseitiger Kenntnis noch keine systematischen CA 19-9-Messungen durchgeführt bzw. über in der großen Mehrzahl der Fälle signifikant erhöhte Messwerten des typischen Tumormarkers CA 19-9 bei Patienten mit systemischen Entzündungen (Sepsis) wurde noch nichts bekannt.

Eine deutliche Erhöhung der CA 19-9-Konzentrationen bei der überwiegenden Zahl von Sepsispatienten wurde erstmals bei den Bestimmungen festgestellt, die im nachfolgenden Versuchsbericht unter Bezugnahme auf zwei Figuren beschrieben werden.

In den Figuren zeigen:
- Fig.1: die Ergebnisse der Bestimmung von CA 19-9 in den Seren von 171 Sepsispatienten im Vergleich mit einer Gruppe von 50 Kontrollpersonen (Blutspendern); und
- Fig.2: die Korrelation der Ergebnisse der CA 19-9-Bestimmungen der 171 Sepsispatienten von Fig.1 mit den Ergebnissen der Procalcitoninbestimmung:

### Versuchsbericht:

In 171 Seren von Sepsis-Patienten, bei denen hohe Werte für den Sepsismarker Procalcitonin (PCT) gefunden worden waren, wurden unter Verwendung eines kommerziellen Assays zur Bestimmung von CA 19-9 (KRYPTOR-CA19-9 der B.R.A.H.M.S Diagnostica GmbH) die Konzentrationen des Tumormarkers CA 19-9 gemessen. Bei 62 % der Seren wurden stark erhöhte CA 19-9 Konzentrationen (mehr als 20 U/ml) gefunden.

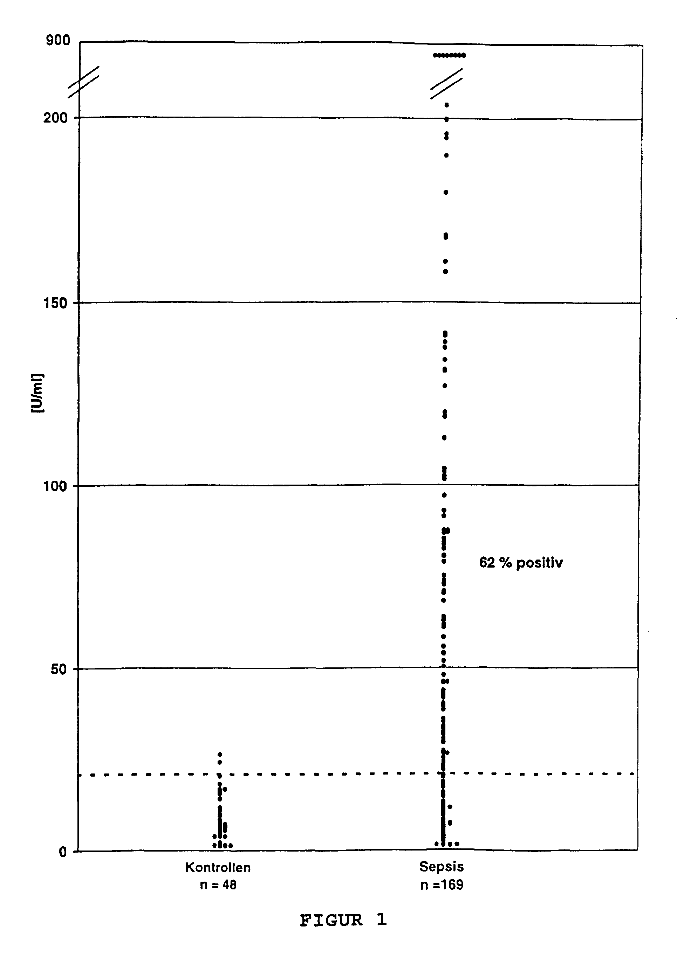

Eine graphische Darstellung der Messergebnisse ist in Figur 1 gezeigt.

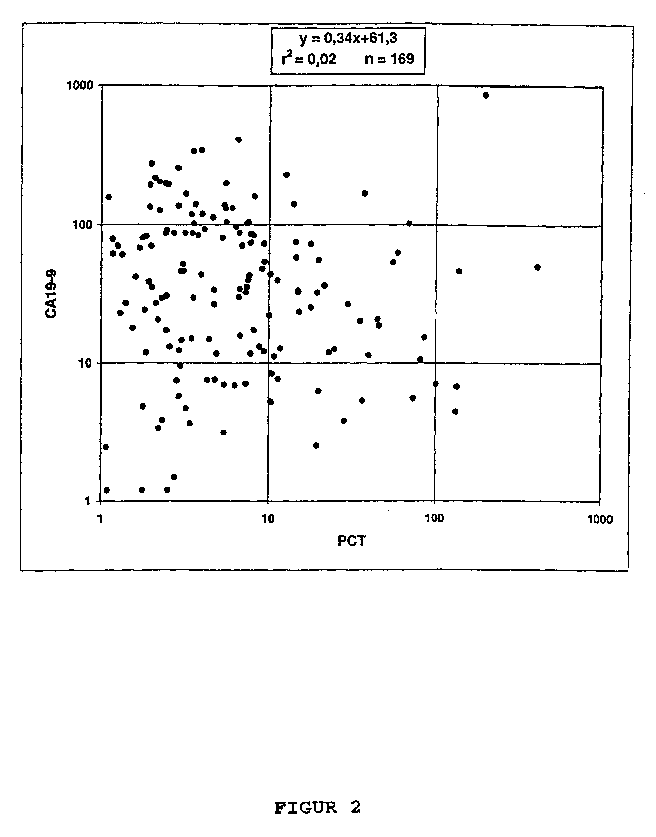

Werden die für individuelle Seren gemessenen Werte für CA 19-9 den für PCT gemessenen Werten gegenübergestellt, ergibt sich keine positive Korrelation in dem Sinne, dass in Seren, in denen hohe PCT Konzentrationen gefunden wurden, auch die höchsten CA 19-9-Werte gefunden werden. Figur 2 zeigt die bei einer solchen Gegenüberstellung gefundenen Korrelationen. Es ist zu erkennen, dass hohe CA 19-9-Werte (oberes Drittel des Diagramms) bei mäßigen PCT-Konzentrationen erhalten werden, und bei sehr hohen PCT-Konzentrationen (rechtes Drittel des Diagramms) mäßige Werte für CA 19-9.

Die weitgehende Unabhängigkeit der Ergebnisse der CA 19-9 Bestimmung von denen der PCT-Bestimmung zeigt, dass trotz der für beide Parameter bei Sepsis erhöhten Werte unterschiedliche Effekte erfasst werden, was bedeutet, dass die Messung beider Parameter mehr Informationen liefert als die Messung nur eines der Parameter.

Die Kombination der Bestimmung von CA 19-9 mit der eines oder mehrerer anderer Sepsismarker bietet sich daher zur Verbesserung der Feindiagnostik von Sepsis und zur Verbesserung der Prognose des Krankheitsverlaufs und zur therapiebegleitenden Verlaufskontrolle bei Sepsispatienten an, wobei die klare Hoffnung besteht, dass die Interpretation der Ergebnisse solcher kombinatorischer Bestimmungen auf der Basis der genauen Auswertung einzelner möglichst vollständig dokumentierter Fälle (mit z.B. Angaben zur Art der Infektion, Grund und Verlauf der Sepsiserkrankung, Kenndaten zum Alter und Geschlecht der Patienten) mit der Anzahl der ausgewerteten Fälle stetig verbessert wird.

Die Bestimmung von CA 19-9 kann dabei nach irgendeinem beliebigen geeigneten Nachweisverfahren erfolgen, wobei jedoch die Bestimmung in einer Körperflüssigkeit eines Patienten auf immundiagnostischem Wege unter Verwendung geeigneter selektiver Antikörper unter praktischen Gesichtspunkten am vorteilhaftesten erscheint. Es stehen bereits kommerzielle Assays zur Bestimmung von CA 19-9 zur Verfügung, die auch im Rahmen der vorliegenden Erfindung genutzt werden können. Dabei ist gegebenenfalls auf eine gute Meßgenauigkeit im für die Sepsisdiagnostik relevanten Messbereich zu achten.

Somit kann die Bestimmung von CA 19-9 zur differentialdiagnostischen Früherkennung und zur Erkennung sowie für die Erstellung einer Verlaufsprognose, zur Beurteilung des Schweregrads und zur therapiebegleitenden Verlaufsbeurteilung von Sepsis erfolgen, indem man bei einem derartigen Verfahren in einer Probe einer biologischen Flüssigkeit eines Patienten den Gehalt von CA 19-9 bestimmt und aus der festgestellten Anwesenheit und/oder Menge von CA 19-9 auf das Vorliegen einer Sepsis schließt und das erhaltene Ergebnis mit dem Schweregrad, dem Fortschritt der Sepsis und/oder dem am stärksten von der Sepsis betroffenen Gewebe oder Organ korreliert und die Behandlungsmöglichkeiten entsprechend wählt und/oder die Behandlungsaussichten abschätzt.

## Patentansprüche

1. In vitro Verfahren zur differentialdiagnostischen Früherkennung und Erkennung, für die Verlaufsprognose und die Beurteilung des Schweregrads und zur therapiebegleitenden Verlaufsbeurteilung von Sepsis, **dadurch gekennzeichnet, dass** man in einer biologischen Flüssigkeit eines Patienten den Parameter CA 19-9 bestimmt und aus dessen Anwesenheit und/oder Menge Schlüsse hinsichtlich des Vorliegens, des zu erwartenden Verlaufs, des Schweregrads und/oder des Erfolgs eingeleiteter Maßnahmen zur Therapie der Sepsis zieht.

2. Verfahren nach Anspruch 1, **dadurch gekennzeichnet, dass** es ein immundiagnostisches Bestimmungsverfahren ist.

3. Verfahren nach Anspruch 1 oder Anspruch 2, **dadurch gekennzeichnet, dass** es im Rahmen einer Multiparameter-Bestimmung durchgeführt wird, bei der gleichzeitig andere Sepsisparameter bestimmt werden, und ein Messergebnis in Form eines Satzes von Messgrößen gewonnen wird, der zur Sepsis-Feindiagnostik ausgewertet wird.

4. Verfahren nach Anspruch 3, **dadruch gekennzeichnet, dass** im Rahmen der Multiparameter-Bestimmung neben CA 19-9 wenigstens ein weiterer Parameter bestimmt wird, der ausgewählt ist aus der Gruppe, die besteht aus Procalcitonin, CA 125, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin und CHP, Peptid-Prohormonen und dem C-reaktiven Protein (CRP).

5. Verfahren nach Anspruch 3 oder 4, **dadurch gekennzeichnet, dass** die Multiparameter-Bestimmung als Simultanbestimmung unter Verwendung einer nach den Verfahren der Chiptechnologie arbeitenden Messvorrichtung erfolgt.

6. Verfahren nach Anspruch 5, **dadurch gekennzeichnet, dass** die Auswertung des komplexen Messergebnisses mit Hilfe eines Computerprogramms erfolgt.

## Claims

1. Method for early differential diagnosis and detection, prognosis and assessment of the severity and therapy-accompanying assessment of the course of sepsis, **characterized in that** the parameter CA 19-9 is determined in a biological fluid of a patient and conclusions are drawn from the presence and/or amount thereof with regard to the presence, the expected course, the severity and/or the success of initiated measures for the treatment of the sepsis.

2. Method according to Claim 1, **characterized in that** it is an immunodiagnostic assay method.

3. Method according to Claim 1 or Claim 2, **characterized in that** it is carried out in the course of a multiparameter determination in which at the same time other sepsis parameters are determined, and a measured result in the form of a set of measured variables, which is evaluated for fine sepsis diagnosis, is obtained.

4. Method according to Claim 3, **characterized in that**, in addition to CA 19-9, at least one further parameter which is selected from the group consisting of procalcitonin, CA 125, S100B, S100A proteins, soluble cytokeratin fragments, in particular CYFRA 21, TPS and/or soluble cytokeratin-1 fragments (sCY1F), the peptides inflammin and CHP, peptide prohormones and the C-reactive protein (CRP) is determined in the course of the multiparameter determination.

5. Method according to Claim 3 or 4, **characterized in that** the multiparameter determination is carried out as a simultaneous determination using a measuring device operating according to the chip technology method.

6. Method according to Claim 5, **characterized in that** the evaluation of the complex measured result is carried out with the aid of a computer program.

## Revendications

1. Procédé in vitro de détection précoce par diagnostic différentiel et d'identification, pour le pronostic de l'évolution, l'évaluation du degré de gravité et l'évaluation de l'évolution d'une sepsie conjointement à une thérapie, **caractérisé en ce que** dans un liquide biologique d'un patient, on détermine le paramètre CA19-9 et, à partir de sa présence et/ou de sa quantité, on tire des conclusions sur la présence, l'évolution attendue, le degré de gravité et/ou le résultat des dispositions prises pour la thérapie de la sepsie.

2. Procédé selon la revendication 1, **caractérisé en ce qu'**il s'agit d'un procédé de détermination par immunodiagnostic.

3. Procédé selon la revendication 1 ou la revendication 2, **caractérisé en ce qu'**il est mis en oeuvre dans le cadre de la détermination de plusieurs paramètres, dans laquelle on détermine en même temps d'autres paramètres de sepsie et l'on recueille le résultat des mesures qui présente la forme d'un ensemble de grandeurs de mesure, qui est évalué pour un diagnostic fin de la sepsie.

4. Procédé selon la revendication 3, **caractérisé en ce que** dans le cadre de la détermination de plusieurs paramètres, on détermine en plus du CA19-9 au moins un autre paramètre qui est sélectionné dans le groupe qui est constitué de la procalcitonine, du CA 125, du S100B, des protéines S100A, des fragments solubles de cytokératine, en particulier le CYFRA 21, le TPS et/ou les fragments solubles de cytokératine-1 (sCY1F), des peptides inflammine et CPH, des prohormones peptidiques et de la protéine C-réactive (CRP).

5. Procédé selon la revendication 3 ou 4, **caractérisé en ce que** la détermination de plusieurs paramètres s'effectue sous la forme d'une détermination simultanée par recours à un dispositif de mesure qui travaille selon le procédé de la technologie des circuits intégrés.

6. Procédé selon la revendication 5, **caractérisé en ce que** l'évaluation du résultat complexe de la mesure s'effectue à l'aide d'un programme informatique.
